# EUROPÄISCHE PATENTSCHRIFT

(11) **EP 2 785 262 B1**
(45) Veröffentlichungstag und Bekanntmachung des Hinweises auf die Patenterteilung: **11.11.2015**
(21) Anmeldenummer: 12790860.6
(22) Anmeldetag: 16.11.2012
(51) Int. Cl.: A61B 17/29

(54) **CHIRURGISCHER HANDGRIFF SOWIE CHIRURGISCHES ROHRSCHAFTINSTRUMENT MIT EINEM CHIRURGISCHEN HANDGRIFF**
SURGICAL HANDGRIP AND SURGICAL TUBULAR SHAFT INSTRUMENT WITH A SURGICAL HANDGRIP
POIGNÉE CHIRURGICALE ET INSTRUMENT CHIRURGICAL À TIGE TUBULAIRE DOTÉ D'UNE POIGNÉE CHIRURGICALE

(30) Priorität: 02.12.2011 DE 102011056003
(43) Veröffentlichungstag der Anmeldung: 08.10.2014
(73) Patentinhaber: Aesculap AG, 78532 Tuttlingen (DE)
(72) Erfinder: HUBER, Christian, 78570 Mühlheim (DE); MORALES, Pedro, 78532 Tuttlingen-Nendingen (DE)
(74) Vertreter: Hoeger, Stellrecht & Partner Patentanwälte mbB
(86) Internationale Anmeldenummer: PCT/EP2012/072865
(87) Internationale Veröffentlichungsnummer: WO 2013/079340

(56) Entgegenhaltungen:
- WO-A1-2009/046490
- DE-A1-102004 025 041
- DE-U1- 9 409 979
- US-A- 5 810 879
- US-A1- 2008 046 003
- US-A1- 2008 064 929
- US-A1- 2008 294 192
- US-A1- 2011 245 864

## Beschreibung

Die Erfindung betrifft einen chirurgischen Handgriff für ein chirurgisches Rohrschaftinstrument nach dem Oberbegriff von Anspruch 1.

Außerdem betrifft die Erfindung ein chirurgisches Rohrschaftinstrument mit einem chirurgischen Handgriff sowie mindestens einem Rohrschaftwerkzeug, das mit dem Handgriff zusammenwirkt.

Ein Handgriff der eingangs genannten Art, wie bekannt US 2011/0245864 A1 aus, kann mit einem Rohrschaftwerkzeug verbunden oder lösbar verbindbar sein zur Ausbildung eines Rohrschaftinstrumentes, beispielsweise eines chirurgischen Greif- oder Schneidinstrumentes. Der Handgriff und das Rohrschaftwerkzeug sind miteinander mittels der Kopplungseinrichtung gekoppelt, wobei der Rohrschaft mit mindestens einem Verbindungselement der Kopplungseinrichtung zusammenwirkt, wobei auch mehrere Verbindungselemente vorgesehen sein können. Im Rohrschaft ist das Kraftübertragungselement, üblicherweise ein Zug- und/oder Druckelement in Gestalt einer Stange oder einer flexiblen Seele, hin- und herbeweglich. Dies ermöglicht eine Relativbewegung des Rohrschaftes und des Kraftübertragungselementes in proximal-distaler Richtung. Diese Relativbewegung kann an einem dem Handgriff gegenüberliegenden Arbeitsende des Rohrschaftes bestimmungsgemäß etwa in eine Relativbewegung von Maulteilen zum Fassen von Körpergewebe oder chirurgischer Instrumente, etwa einer Nadel, umgesetzt werden, oder in eine Bewegung eines chirurgischen Trennelementes zum Durchschneiden von Körpergewebe.

"Proximal" und "distal" sind vorliegend als auf einen den Handgriff und das Rohrschaftinstrument handhabenden Benutzer bezogen aufzufassen. Der Benutzer wirkt von proximal auf den Handgriff ein, und ausgehend vom Handgriff erstreckt sich das Rohrschaftwerkzeug in distaler Richtung, wobei das Arbeitsende des Rohrschaftwerkzeuges üblicherweise an dessen distalem Ende angeordnet ist.

Zum Einwirken auf das Kraftübertragungselement umfasst die Kopplungseinrichtung das Kopplungselement, das mit Hilfe der Griffeinrichtung relativ zum mit dem Rohrschaft verbundenen Verbindungselement beweglich ist, so dass damit auch die Relativbewegung des Kraftübertragungselementes und des Rohrschaftes erzielt werden kann. Auf das Kopplungselement kann mit der Griffeinrichtung eingewirkt werden, die von der Nichtbetätigungs- in zumindest eine Betätigungsstellung überführt wird, so dass dadurch das Kopplungselement von einer Nichtbetätigungs- in zumindest eine Betätigungsposition überführt wird.

Aufgabe der vorliegenden Erfindung ist es, einen Handgriff der eingangs genannten Art bereitzustellen, mit dem auf handhabungsfreundlichere Weise auf ein mit dem Handgriff verbundenes oder verbindbares Rohrschaftwerkzeug eingewirkt werden kann.

Diese Aufgabe wird bei einem gattungsgemäßen Handgriff erfindungsgemäß durch die Merkmale des kennzeichnenden Teils von Anspruch 1 gelöst.

Durch den Führungskörper der Führungseinrichtung kann dem Kopplungselement beim Überführen von der Nichtbetätigungsposition in die zumindest eine Betätigungsposition sowie bevorzugt auch umgekehrt eine definierte Bewegungsrichtung vorgegeben werden. Die Bewegungsrichtung erfolgt längs der Griffachse, die typischerweise proximal-distal ausgerichtet ist und insbesondere eine proximal-distale Achse definiert. Das Rohrschaftwerkzeug kann zumindest an seinem proximalen Ende so ausgebildet sein, dass es eine Werkzeugachse definiert, längs derer das Kraftübertragungselement relativ zum Rohrschaft hin- und herbeweglich ist. Wenn die Werkzeugachse mit der Griffachse in Übereinstimmung gebracht oder koaxial relativ zu dieser ausgerichtet werden kann, ist dadurch die Möglichkeit gegeben, das Kraftübertragungselement längs der Werkzeugachse und insbesondere längs der Griffachse zu führen. Der erfindungsgemäße Handgriff erlaubt es somit, das Kopplungselement und ein mit diesem zusammenwirkendes Kraftübertragungselement axial zu führen. Dies gibt die Möglichkeit, beim Betätigen der Griffeinrichtung unabhängig von deren Stellung eine rein axiale Bewegung des Kraftübertragungselementes mittels des Kopplungselementes herbeizuführen, ohne dass Querkräfte auf das Kraftübertragungselement einwirken. Durch die Vermeidung von Querkräften auf das Kraftübertragungselement wird zum Einen eine vom Benutzer an der Griffeinrichtung aufbringbare Betätigungskraft verringert. Zugleich kann die Betätigungskraft mittels des Handgriffes besser auf das Rohrschaftwerkzeug eingeleitet werden, was ein feinfühligeres Arbeiten ermöglicht. Im Ergebnis ist der erfindungsgemäße Handgriff und damit ein dieses aufweisendes Rohrschaftwerkzeug für einen Benutzer auf einfachere Weise handhabbar. Zum Anderen kann der Verschleiß des Kraftübertragungselementes durch die Vermeidung von Querkräften verringert werden und dadurch die Lebensdauer eines mit dem Handgriff zusammenwirkenden Rohrschaftwerkzeuges erhöht werden.

Der Führungskörper definiert die Griffachse, und das Kopplungselement ist beim Überführen von der Nichtbetätigungsposition in die mindestens eine Betätigungsposition vom Führungskörper verschieblich geführt. Dies ermöglicht es, eine Führung des Kopplungselementes auf konstruktiv einfache und zuverlässige Weise sicherzustellen.

Der Führungskörper ist als axiale Hülse ausgestaltet, in der das Kopplungselement verschieblich gelagert ist. Dies erlaubt eine konstruktiv einfache und zugleich kompakte Bauform des Handgriffes. Die Hülse ist z.B. zumindest abschnittsweise zylindrisch ausgestaltet. An einem distalen Ende kann sie geöffnet sein zum Einführen des Rohrschaftes und des Kraftübertragungselementes. Im Inneren der Hülse ist das Kopplungselement günstigerweise formschlüssig aufgenommen und dadurch längs einer Achse der Hülse, die die Griffachse definiert, verschieblich gelagert.

Von Vorteil ist es, dass die Hülse eine axiale schlitzförmige Durchbrechung längs zumindest eines Abschnittes eines vom Kopplungselement relativ zur Hülse beim Überführen von der Nichtbetätigungsposition in die zumindest eine Betätigungsposition und umgekehrt zurücklegbaren Verschiebeweges aufweist. Durch die schlitzförmige Durchbrechung kann ein Wirkelement des Handgriffes hindurch greifen, mit dem das Kopplungselement mit der Griffeinrichtung in Wirkverbindung steht. Dadurch kann eine Betätigungskraft des Benutzers von der Außenseite durch die Hülse hindurch auf das Kopplungselement übertragen werden. Dies ermöglicht eine kompakte Bauform des Handgriffes. Weiter kann vorgesehen sein, dass das Wirkelement die Durchbrechung formschlüssig oder im Wesentlichen formschlüssig durchgreift. Dies erlaubt es, das Wirkelement mittels der Hülse parallel zur Griffachse zu führen und dadurch die Bewegung des Kopplungselementes noch definierter zu gestalten. Es kann mehr als nur eine schlitzförmige Durchbrechung und/oder ein Wirkelement vorgesehen sein. Zum Beispiel umfasst die Griffeinrichtung zwei Griffelemente, von denen jedes über ein Wirkelement mit dem Kopplungselement in Wirkverbindung steht. Jedes der Wirkelemente kann eine der Durchbrechungen durchgreifen.

Die Hülse weist eine fensterförmige Durchbrechung auf, die in Umfangsrichtung der Griffachse einen größeren Winkelbereich überdeckt als die schlitzförmige Durchbrechung. Die fensterförmige Durchbrechung erlaubt es beispielsweise, darauf wird nachfolgend noch eingegangen, dass das Kopplungselement relativ zum Führungskörper quer oder schräg zur Griffachse ausgerückt werden kann und dabei teilweise in die Durchbrechung eingreift oder diese durchgreift. Dies kann insbesondere vorgesehen sein, wenn das Kopplungselement mit dem Kraftübertragungselement gekoppelt oder von diesem entkoppelt werden soll. Es zeigt sich in der Praxis, dass der Hülse und dem Handgriff dadurch eine kompakte Bauform verliehen werden können. In axialer Richtung ist die fensterförmige Durchbrechung günstigerweise deutlich kürzer ausgestaltet als die schlitzförmige Durchbrechung.

Günstig ist es, wenn das Kopplungselement vom Führungskörper längs des gesamten Bewegungsumfanges des Kopplungselementes und des Führungskörpers relativ zueinander geführt ist. Dadurch kann dem Kopplungselement, und damit auch dem Kraftübertragungselement, längs des gesamten Bewegungsumfangs relativ zum Führungskörper eine klar definierte Bewegungsrichtung vorgegeben werden.

Es kann vorgesehen sein, dass der Führungskörper die Wirkverbindung des Kopplungselementes mit der Griffeinrichtung bereitstellt und das Kopplungselement und die Griffeinrichtung zu diesem Zweck miteinander verbindet.

Bei einer Umsetzung des erfindungsgemäßen Handgriffes in der Praxis erweist es sich als vorteilhaft zur Erzielung einer konstruktiv einfachen Ausgestaltung und kompakten Bauform, wenn die fensterförmige Durchbrechung distalseitig der schlitzförmigen Durchbrechung angeordnet ist.

Die Hülse und damit der Handgriff lassen sich auf einfachere Weise herstellen, wenn die schlitzförmige Durchbrechung und die fensterförmige Durchbrechung in Umfangsrichtung der Griffachse auf derselben Seite der Hülse angeordnet sind.

Aus demselben Grund ist es von Vorteil, wenn die schlitzförmige Durchbrechung und die fensterförmige Durchbrechung ineinander münden.

Allgemein ist es von Vorteil, wenn der Handgriff ein elastisches Rückstellelement umfasst, entgegen dessen Wirkung das Kopplungselement von der Nichtbetätigungsposition in die zumindest eine Betätigungsposition überführbar ist. Dies erleichtert einem Benutzer die Handhabung des Handgriffes.

Insbesondere kann vorgesehen sein, dass der Handgriff ein in der Hülse angeordnetes elastisches Rückstellelement umfasst, entgegen dessen Wirkung das Kopplungselement von der Nichtbetätigungsposition in die zumindest eine Betätigungsposition verschiebbar ist. Die Anordnung des Rückstellelementes in der Hülse ermöglicht einen kompakten Aufbau des Handgriffes.

Zur Erzielung desselben Vorteils ist das Rückstellelement vorzugsweise proximalseitig des Kopplungselementes angeordnet und stützt sich proximalseitig an einem von der Hülse umfassten oder an dieser festgelegten Stützelement ab. Distalseitig kann sich das Rückstellelement am ersten Kopplungselement abstützen. Das Rückstellelement kann damit axial zwischen dem distalseitig angeordneten Kopplungselement und dem proximalseitig angeordneten Stützelement angeordnet, was dem Handgriff einen kompakten Aufbau verleiht. Das Stützelement kann beispielsweise eine proximalseitige Wand der Hülse sein oder ein die Hülse proximalseitig verschließendes Verschlusselement, das mit der Hülse verbunden ist.

Günstig ist es, wenn die Griffeinrichtung ein erstes Griffelement umfasst, das um eine quer zur Griffachse ausgerichtete Schwenkachse am Führungskörper oder an einem mit dem Führungskörper verbundenen Lagerkörper schwenkbar gelagert ist. Die schwenkbare Lagerung des ersten Griffelementes erlaubt dessen klar definierte und handhabungsfreundliche Bewegung relativ zum Führungskörper oder zum Lagerkörper. Zur Erzielung eines kompakten Aufbaus des Handgriffes ist das erste Griffelement günstigerweise unmittelbar am Führungskörper schwenkbar gelagert. "Quer zur Griffachse" bedeutet vorliegend in einer Ebene, senkrecht zu der die Griffachse ausgerichtet ist.

Bei einer Umsetzung des Handgriffes in der Praxis erweist es sich für dessen Handhabung als vorteilhaft, wenn das erste Griffelement mit einem proximalen Ende schwenkbar gelagert ist und mit einem distalen Ende relativ zum Führungskörper schwenkbar ist. Dadurch kann das erste Griffelement vom Benutzer auf einfachere Weise ergriffen und betätigt werden.

Vorzugsweise ist das erste Griffelement an einem proximalen oder nahe einem proximalen Ende des Handgriffes schwenkbar gelagert, denn dies erlaubt es, dem Handgriff eine kompakte Konstruktion zu verleihen.

Bevorzugt ist das erste Griffelement ausgehend von einer abgespreizten Stellung relativ zum Führungskörper, die es in der Nichtbetätigungsstellung einnimmt, in eine angenäherte Stellung relativ zum Führungskörper, die es in der mindestens einen Betätigungsstellung einnimmt, und umgekehrt überführbar. Zum Betätigen des Handgriffs kann das Griffelement von der abgespreizten Stellung in die angenäherte Stellung unter Verschwenken relativ zum Führungskörper überführt werden. Dies erleichtert einem Benutzer die Handhabung des Handgriffes.

Günstig ist es, wenn das erste Griffelement in zumindest einer Betätigungsstellung fixierbar ist, denn dies gibt die Möglichkeit, auch das Kopplungselement und damit das Kraftübertragungselement in einer Stellung zu fixieren. Dies ist beispielsweise günstig, wenn am Arbeitsende des Rohrschaftwerkzeuges Maulteile zum Fassen von Körpergewebe oder eines chirurgischen Instrumentes wie etwa einer Nadel angeordnet sind.

Beispielsweise ist das erste Griffelement in der Betätigungsstellung mit dem Führungskörper oder dem Lagerkörper verrastbar. Dies verleiht dem Handgriff eine konstruktiv einfache Ausgestaltung. Zudem kann vorgesehen sein, dass das erste Griffelement in der verrasteten Stellung eine Anlagestellung relativ zum Führungskörper oder Lagerkörper aufweist, in der es am Führungskörper oder am Lagerkörper anliegt und relativ zu diesem eine klar definierte Position einnimmt.

Wie eingangs erwähnt, steht das Kopplungselement mit der Griffeinrichtung in Wirkverbindung. Bei einer konstruktiv einfachen und zuverlässig arbeitenden Umsetzung des Handgriffes in der Praxis erweist es sich als vorteilhaft, wenn der Handgriff ein Gelenkelement umfasst, das an einem ersten Gelenk um eine quer zur Griffachse ausgerichtete erste Gelenkachse schwenkbar mit dem ersten Griffelement verbunden ist und an einem zweiten Gelenk um eine quer zur Griffachse ausgerichtete zweite Gelenkachse schwenkbar mit dem Kopplungselement verbunden ist. Das Gelenkelement kann auf diese Weise einen Gelenkhebel ausbilden, um eine Betätigungskraft des Benutzers vom ersten Griffelement auf das Kopplungselement zu übertragen. Insbesondere kann das Gelenkelement das vorstehend erwähnte Wirkelement bilden, das eine schlitzförmige Durchbrechung im als Hülse ausgestalteten Führungskörper durchgreifen kann.

Vorzugsweise spannt die zweite Gelenkachse eine Ebene mit der Griffachse auf. Das zweite Gelenk kann dadurch längs der oder parallel zur Griffachse bewegt werden, wenn die Griffeinrichtung betätigt wird, was sich in der Praxis als vorteilhaft für die Funktion des Handgriffes erweist.

Vorteilhafterweise sind das erste Gelenk und/oder das zweite Gelenk distalseitig der Schwenkachse angeordnet, insbesondere wenn das erste Griffelement mit einem proximalen Ende am Führungskörper oder am Lagerkörper verschwenkbar gelagert ist.

Zur Erzielung eines kompakten Aufbaus des Handgriffes, insbesondere in Kombination mit der zuletzt erwähnten vorteilhaften Ausführungsform, ist das zweite Gelenk beim Überführen des ersten Griffelementes von der Nichtbetätigungsstellung in die zumindest eine Betätigungsstellung und umgekehrt proximalseitig des ersten Gelenkes angeordnet.

Die Griffeinrichtung umfasst bevorzugt ein zweites Griffelement, um dem Benutzer die Handhabung des Handgriffes zu erleichtern. Es kann vorzugsweise vorgesehen sein, dass das zweite Griffelement unbeweglich am Führungskörper oder am Lagerkörper festgelegt ist, denn dadurch kann dem Handgriff eine einfachere konstruktive Ausgestaltung verliehen werden.

Das zweite Griffelement kann auch beweglich sein und beispielsweise am Führungskörper oder am Lagerkörper um eine quer zur Griffachse ausgerichtete Schwenkachse schwenkbar gelagert sein. Ferner kann das zweite Griffelement mit dem Kopplungselement durch ein Wirkelement oder Gelenkelement in Wirkverbindung stehen, um eine Betätigungskraft auf das Kopplungselement zu übertragen.

Bei einer Umsetzung des Handgriffes in der Praxis erweist es sich als günstig, wenn das erste Griffelement und/oder das zweite Griffelement schalenförmig ausgestaltet sind und den Führungskörper, insbesondere hülsenartig, in Umfangsrichtung der Griffachse zumindest bereichsweise umgeben. Die Griffelemente sind beispielsweise an zwei einander gegenüberliegenden Seiten der Griffachse angeordnet und können vom Benutzer mit der Handfläche auf einfachere Weise ergriffen und betätigt werden.

Weiter erweist es sich als vorteilhaft, wenn das erste Griffelement und das zweite Griffelement jeweils als axial erstreckte halbzylindrische oder im Wesentlichen halbzylindrische Schalen ausgestaltet sind, die den Führungskörper zwischen sich aufnehmen.

Von Vorteil ist es, wenn das Kopplungselement ein Bewegungsglied umfasst, das vom Führungskörper beweglich und insbesondere verschiebbar geführt ist, sowie ein mit dem Bewegungsglied gekoppeltes Aufnahmeglied, das eine Aufnahme für das Kraftübertragungselement umfasst. Es kann hierbei vorgesehen sein, dass auch das Aufnahmeglied mit der Aufnahme für das Kraftübertragungselement, zumindest abschnittsweise längs der Griffachse, vom Führungskörper geführt ist. Das Bewegungs- und speziell Verschiebeglied kann einstückig mit dem Aufnahmeglied verbunden sein und/oder mit dem Führungskörper verbunden sein.

Weiter ist es günstig, wenn das Kopplungselement von einer Kopplungsstellung in eine Entkopplungsstellung und umgekehrt überführbar ist, wobei in der Kopplungsstellung eine Einführöffnung der Aufnahme für das Kraftübertragungselement axial blockiert und in der Entkopplungsstellung die Einführöffnung axial freigegeben ist. Dies erleichtert es, das Kraftübertragungselement mit dem Kopplungselement zu verbinden und/oder von diesem zu lösen, weswegen die Entkopplungsstellung auch als Ankopplungsstellung bezeichnet werden kann. Zu diesem Zweck kann das Kopplungselement in die Entkopplungsstellung überführt und dadurch die Aufnahme für das Kraftübertragungselement axial freigegeben werden. Nimmt das Kopplungselement die Kopplungsstellung ein, ist die Einführöffnung axial blockiert, so dass das Kraftübertragungselement nicht mit dem Kopplungselement verbunden und/oder von diesem gelöst werden kann.

Bevorzugt ist das Aufnahmeglied distalseitig des Bewegungsglieds angeordnet, denn dies erlaubt es, dem Handgriff eine konstruktiv einfachere Ausgestaltung zu verleihen.

Bei einer konstruktiv einfachen Ausgestaltung des Handgriffes weist die Aufnahme ein im Aufnahmeglied gebildetes, quer zur Griffachse orientiertes Sackloch auf mit einer in axialer Richtung geschlitzten Seitenwand. Eine derartige Aufnahme eignet sich insbesondere zum Aufnehmen einer im Kraftübertragungselement angeordneten Gelenkkugel. Die Gelenkkugel kann durch die Einführöffnung hindurch in das Sackloch eingeführt werden. Eine Stange oder eine Seele, an der die Gelenkkugel festgelegt ist, kann die geschlitzte Seitenwand der Aufnahme durchgreifen und aus der Aufnahme herausgeführt werden.

Zur Handhabung des Handgriffes erweist es sich als günstig, wenn das Bewegungsglied und das Aufnahmeglied relativ zueinander beweglich sind zum Überführen des Kopplungselementes von der Kopplungsstellung in die Entkopplungsstellung und umgekehrt.

Insbesondere ist es von Vorteil, wenn das Aufnahmeglied und das Bewegungsglied relativ zueinander um eine quer zur Griffachse ausgerichtete Ausrückachse schwenkbar sind. Beispielsweise ist dadurch die Möglichkeit gegeben, allein das Aufnahmeglied relativ zum Bewegungsglied zu verschwenken, welches eine ortsfeste Stellung relativ zum Führungskörper beibehalten kann.

Für eine zuverlässige Funktion des Handgriffes erweist es sich in der Praxis als vorteilhaft, wenn die Ausrückachse und die Griffachse eine Ebene definieren.

Günstig ist es, wenn das Aufnahmeglied zum Überführen des Kopplungselementes von der Kopplungsstellung in die Entkopplungsstellung und umgekehrt von der Griffachse weg bzw. auf die Griffachse zu verschwenkbar ist. Wird das Aufnahmeglied von der Griffachse weg geschwenkt, kann dadurch die Aufnahme so in Richtung der Griffachse verschwenkt werden, dass sie axial freigegeben ist und das Kopplungselement die Entkopplungsstellung einnimmt. Wird das Aufnahmeglied auf die Griffachse zu verschwenkt, kann die Einführöffnung von der Griffachse weg verschwenkt und in Abstand zu dieser gebracht werden, und dadurch die Aufnahme so axial blockiert werden, dass das Kopplungselement die Kopplungsstellung einnimmt.

Beim Verschwenken des Aufnahmegliedes relativ zur Griffachse ist es günstig, wenn der Führungskörper in Gestalt einer Hülse die vorstehend erwähnte fensterförmige Durchbrechung aufweist, in die das Aufnahmeglied eingreifen oder die es durchgreifen kann. Wie bereits erwähnt, kann dadurch dem Handgriff eine kompakte Bauform verliehen werden.

Für eine vereinfachte Handhabung des Handgriffes ist es von Vorteil, wenn das Kopplungselement zum Überführen von der Kopplungsstellung in die Entkopplungsstellung längs der Griffachse beweglich und insbesondere verschieblich ist.

Vorzugsweise ist das Kopplungselement zum Überführen von der Kopplungsstellung in die Entkopplungsstellung in Gegenrichtung der Bewegungs- und insbesondere Verschieberichtung des Kopplungselementes von der Nichtbetätigungsposition in die mindestens eine Betätigungsposition beweglich und insbesondere verschiebbar. Dadurch lässt sich vermeiden, dass das Kopplungselement beim Betätigen des Handgriffes nicht unbeabsichtigt in die Entkopplungsstellung überführt wird.

In entsprechender Weise ist es vorteilhaft, wenn die Griffeinrichtung zum Überführen des Kopplungselementes von der Kopplungsstellung in die Entkopplungsstellung in Gegenrichtung der Überführung der Griffeinrichtung von der Nichtbetätigungsstellung in die mindestens eine Betätigungsstellung überführbar ist.

Beim Überführen des Kopplungselementes von der Kopplungs- in die Entkopplungsstellung und/oder dem Überführen der Griffeinrichtung ist bevorzugt unterstützend das vorstehend erwähnte elastische Rückstellelement wirksam.

Bevorzugt umfasst die Führungseinrichtung Führungsglieder zum Führen des Kopplungselementes beim Überführen von der Entkopplungsstellung in die Kopplungsstellung und/oder umgekehrt. Dadurch kann das Kopplungselement in definierter Weise von der Kopplungs- in die Entkopplungsstellung und/oder umgekehrt überführt und dadurch die Handhabung des Handgriffes erleichtert werden.

Von Vorteil ist es, wenn der Führungskörper mindestens ein Führungsglied umfasst oder ausbildet oder mindestens ein Führungsglied an diesem angeordnet ist, das mit mindestens einem vom Kopplungselement umfassten oder ausgebildeten Führungsglied zusammenwirkt. Durch das Vorsehen eines Führungsgliedes am Führungskörper kann dem Handgriff ein konstruktiv einfacher und kompakter Aufbau verliehen werden. Das mit dem Führungsglied zusammenwirkende Führungsglied ist bevorzugt am Aufnahmeglied angeordnet, welches günstigerweise relativ zum Bewegungsglied um die Ausrückachse schwenkbar ist.

Bei einer Umsetzung des Handgriffes in der Praxis erweist es sich als günstig, wenn ein Führungsglied als quer zur Griffachse ausgerichteter Führungsstift, das mit ihm zusammenwirkende Führungsglied als schräg zur Griffachse ausgerichtete Führungsfläche ausgebildet ist und/oder wenn zusammenwirkende Führungsglieder als aneinander gleitende, schräg zur Griffachse ausgerichtete Führungsflächen ausgebildet sind. Beispielsweise kann zum Führen des Kopplungselementes von der Kopplungsstellung in die Entkopplungsstellung ein Führungsstift mit einer Führungsfläche zusammenwirken und zum Überführen von der Entkopplungsstellung in die Kopplungsstellung zwei aneinander gleitende Führungsflächen zusammenwirken. Der Führungsstift kann am Führungskörper angeordnet sein oder von diesem umfasst oder ausgebildet werden oder am Aufnahmeglied. In entsprechender Weise können die Führungsflächen Führungsflächen des Führungskörpers oder des Aufnahmegliedes sein.

Weiter erweist es sich zum definierten Überführen des Kopplungselementes von der Kopplungsstellung in die Entkopplungsstellung als vorteilhaft, wenn die Führungseinrichtung Anschlagglieder umfasst zum Begrenzen des Bewegungs- und insbesondere Verschiebeweges des Kopplungselementes längs der Griffachse beim Überführen von der Kopplungsstellung in die Entkopplungsstellung.

Bei einer Umsetzung des Handgriffes erweist es sich zur Erzielung eines konstruktiv einfachen und kompakten Aufbaus als günstig, wenn der Führungskörper mindestens ein Anschlagglied umfasst oder ausbildet oder ein solches am Führungskörper angeordnet ist, welches mit mindestens einem vom Kopplungselement umfassten oder ausgebildeten Anschlagglied zusammenwirkt. Beispielsweise kann ein Anschlagglied als quer zur Griffachse ausgerichteter Anschlagstift ausgebildet sein, das mit ihm zusammenwirkende Anschlagglied als quer zur Griffachse ausgerichteter Absatz am Kopplungselement bzw. am Führungskörper. Der Anschlagstift kann am Führungskörper oder am Kopplungselement angeordnet sein und dementsprechend der Absatz am Kopplungselement oder am Führungskörper. Beispielsweise ist der Absatz am Bewegungsglied angeordnet.

Bei einer konstruktiv einfachen Ausgestaltung, insbesondere bei einem Führungskörper in Gestalt einer Hülse, ist es günstig, wenn das Kopplungselement zumindest abschnittsweise zylindrisch oder im Wesentlichen zylindrisch ausgestaltet und koaxial zur Griffachse ausgerichtet ist.

Ferner kann vorgesehen, dass das Kopplungselement zumindest abschnittsweise axial geschlitzt ausgebildet ist. In den axialen Schlitz kann z.B. das vorstehend erwähnte Gelenkelement eingreifen, mit dem das Kopplungselement mit der Griffeinrichtung gekoppelt sein kann.

Wie bereits erwähnt, betrifft die Erfindung auch ein chirurgisches Rohrschaftinstrument. Ein erfindungsgemäßes chirurgisches Rohrschaftinstrument umfasst einen der vorstehend erläuterten Handgriffe sowie mindestens ein Rohrschaftwerkzeug, das mit dem Handgriff zusammenwirkt. Dadurch lassen sich die bereits im Zusammenhang mit dem erfindungsgemäßen Handgriff sowie vorteilhafter Ausführungsformen davon erläuterten Vorteile ebenfalls erzielen, so dass diesbezüglich auf voranstehende Erläuterungen verwiesen wird.

Zumindest im Bereich des proximalen Endes kann das mindestens eine Rohrschaftwerkzeug eine Werkzeugachse definieren, die eine Achse des Kraftübertragungselementes und des Rohrschafts des Rohrschaftwerkzeuges ist. Die Werkzeugachse kann in Übereinstimmung mit der Griffachse gebracht oder koaxial zu dieser ausgerichtet werden.

Bei den Rohrschaftwerkzeugen kann es sich um verschiedenartige Rohrschaftwerkzeuge handeln, etwa solche mit Maulteilen am Arbeitsende oder mit einer chirurgischen Trennvorrichtung, die jeweils auch in unterschiedlichen Größen und/oder Konfigurationen vorliegen können.

Insbesondere bei Vorhandensein mehr als nur eines Rohrschaftwerkzeuges ist es günstig, wenn das mindestens eine Rohrschaftwerkzeug lösbar mit dem Handgriff verbindbar ist.

Die nachfolgende Beschreibung bevorzugter Ausführungsformen der Erfindung dient im Zusammenhang mit der Zeichnung der näheren Erläuterung der Erfindung. Es zeigen:
- Figur 1:: Eine perspektivische Ansicht eines erfindungsgemäßen chirurgischen Handgriffs;
- Figur 2:: eine Längsschnittansicht eines erfindungsgemäßen Rohrschaftwerkzeuges, umfassend den Handgriff aus Figur 1 sowie ein mit diesem verbundenes Rohrschaftwerkzeug, wobei ein Kopplungselement des Handgriffs eine Entkopplungsstellung einnimmt;
- Figur 3:: eine vergrößerte Darstellung von Detail A in Figur 2;
- Figur 4:: eine Längsschnittansicht des Handgriffs aus Figur 1, wobei das Kopplungselement eine Kopplungsstellung einnimmt und sich in einer Nichtbetätigungsposition befindet und
- Figur 5:: eine Längsschnittansicht des Handgriffs aus Figur 1 mit dem Kopplungselement in der Kopplungsstellung und in einer Betätigungsposition.

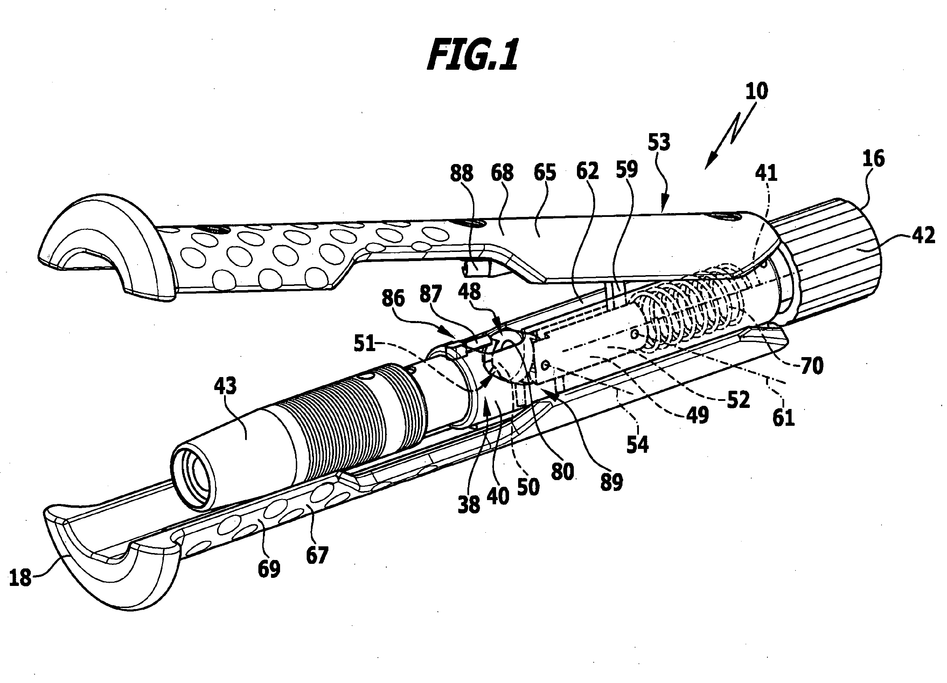

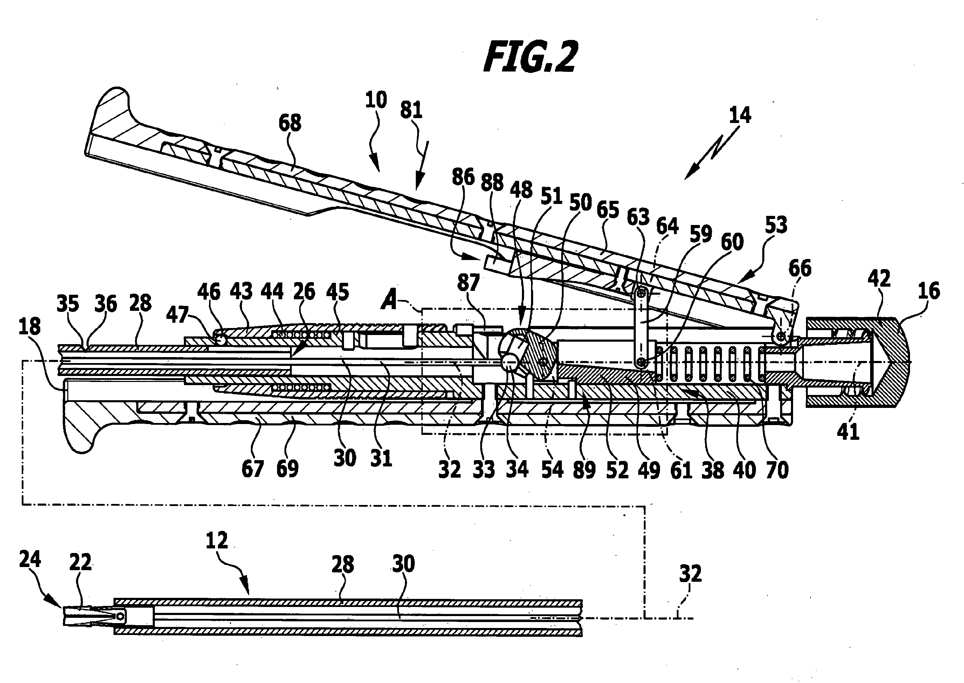

Figur 1 zeigt in perspektivischer Darstellung eine bevorzugte Ausführungsform eines insgesamt mit dem Bezugszeichen 10 belegten chirurgischen Handgriffes. Der Handgriff 10 kann mit einem in Figur 2 teilweise dargestellten Rohrschaftwerkzeug 12 zusammenwirken, um eine bevorzugte Ausführungsform eines mit dem Bezugszeichen 14 belegten erfindungsgemäßen chirurgischen Rohrschaftinstrumentes auszubilden. Der Handgriff 10 zeichnet sich durch einen nachfolgend erläuterten konstruktiv einfachen, kompakten und handhabungsfreundlichen Aufbau aus.

Der Handgriff 10 umfasst einen im Wesentlichen längs erstreckten, schlanken Aufbau mit einem proximalen Ende 16 und einem distalen Ende 18, wobei "proximal" und "distal" als auf einen den Handgriff 10 einsetzenden Benutzer bezogen aufzufassen sind. Der Benutzer greift den Handgriff 10 von proximal und wirkt in distaler Richtung auf einen in der Zeichnung nicht dargestellten Patienten ein. Die proximal-distale Richtung gibt die Arbeitsrichtung vor, entlang derer sich im bestimmungsgemäßen Gebrauch des Rohrschaftinstrumentes 14 das Rohrschaftwerkzeug 12 erstreckt.

Das Rohrschaftwerkzeug 12 umfasst ein distales Ende 22, das zugleich ein Arbeitsende des Rohrschaftinstrumentes 14 ist, und an dem ein chirurgisches Klemmelement in Form eines zweiteiligen Maulteils 24 zum Fassen von Körpergewebe oder eines chirurgischen Instrumentes, etwa einer Nadel, angeordnet ist. Mit einem proximalen Ende 26 ist das Rohrschaftwerkzeug 12 lösbar mit dem Handgriff 10 verbindbar.

Das Rohrschaftwerkzeug 12 weist in an sich bekannter Weise einen Rohrschaft 28 auf sowie ein in diesem proximal-distal hin- und her bewegliches Kraftübertragungselement 30 in Gestalt einer Stange 31, die sowohl auf Zug als auch auf Druck reagiert, wobei der Rohrschaft 28 weiter von einer Außenhülse oder einem Außenrohr umgeben sein kann. Der Rohrschaft 28 und die Stange 31 definieren eine Werkzeugachse 32, koaxial zu der sie ausgerichtet sind. Mit einem distalen Ende der Stange kann in an sich bekannter Weise auf das Maulteil 24 eingewirkt werden, um dieses zu schließen und zu öffnen, um Körpergewebe oder das chirurgisches Instrument wie etwa eine Nadel zu fassen bzw. freizugeben. Dies ist an sich bekannt, so dass die diesbezügliche zeichnerische Darstellung im Bereich des distalen Endes 22 nur schematisch ist. Die Übertragung der Kraft von der Stange 31 auf das Maulteil 24 ist derart, dass das Maulteil 24 auf Zug geschlossen wird, d. h. wenn die Stange 31 in proximaler Richtung relativ zum Rohrschaft 28 bewegt wird.

Proximalseitig weist die Stange 31 ein Kopplungselement 33 in Gestalt einer Kugel 34 auf, die proximalseitig außerhalb des Rohrschaftes 28 angeordnet ist.

Der Rohrschaft 28 umfasst im Abstand zum proximalen Ende 26 drei Verbindungselemente in Gestalt von Ausnehmungen, die bezüglich der Werkzeugachse 32 jeweils im Winkel von 120° zueinander angeordnet sind. Hiervon ist nur ein Verbindungselement 35 in Form der Ausnehmung 36 in Figur 2 zu sehen. Die Verbindungselemente sowie das Kopplungselement 33 dienen zum Ankoppeln des Rohrschaftwerkzeuges 12 an den Handgriff 10.

Der Handgriff 10 weist einen Grundkörper auf in Gestalt eines Führungskörpers 38. Der Führungskörper 38 ist ausgestaltet als eine im Wesentlichen zylindrische Hülse 40, die eine Griffachse 41 des Handgriffes 10 definiert. Proximalseitig ist die Hülse 40 mittels eines Verschlusselementes in Gestalt eines Stopfens 42 lösbar verschlossen. Distalseitig ist die Hülse 40 geöffnet, so dass der Rohrschaft 28 und das Kraftübertragungselement 30 zum Koppeln mit dem Handgriff 10 eingeführt werden können.

Der Handgriff 10, insbesondere dessen Hülse 40, und das Rohrschaftwerkzeug 12 sind hinsichtlich ihrer Abmessungen aufeinander abgestimmt. Dabei ist der Innendurchmesser der Hülse 40 so gewählt, dass der Rohrschaft 28 spielfrei in das distale Ende der Hülse 40 eingeführt werden kann. Der Rohrschaft 28 und die Stange 31 sind dadurch koaxial zur Hülse 40 ausgerichtet, und die Werkzeugachse 32 kann mit der Griffachse 41 in Übereinstimmung gebracht werden.

Über das distale Ende der Hülse 40 ist ein Haltekörper geschoben in Gestalt einer Außenhülse 43, die sich entgegen der Wirkung eines elastischen Elementes in Gestalt einer Schraubenfeder 44 an einem Stützelement der Hülse 40 in Gestalt einer Ringschulter 45 abstützt. Die Außenhülse 43 wird dabei von der Schraubenfeder 44 in distaler Richtung mit einer Rückstellkraft beaufschlagt, und sie dient auf diese Weise zur Fixierung von Verbindungselementen des Handgriffes 10 zum Zusammenwirken mit dem Rohrschaft 28. Die Verbindungselemente, hiervon ist nur ein Verbindungselement 46 in Gestalt einer Rastkugel 47 gezeigt, werden von der Außenhülse 43 radial umgeben und können durch Öffnungen der Hülse 40 hindurch in Eingriff mit den drei Ausnehmungen 36 des Rohrschafts 28 gebracht werden. Der in die Hülse 40 eingeführte Rohrschaft 28 kann dadurch an der Hülse 40 fixiert werden. Zum Verbinden und Lösen des Rohrschafts 28 kann die Außenhülse 43 entgegen der Wirkung der Schraubenfeder 44 in proximaler Richtung verschoben werden, so dass die Rastkugeln 47 einfacher in bzw. außer Eingriff mit den Ausnehmungen 36 gebracht werden können.

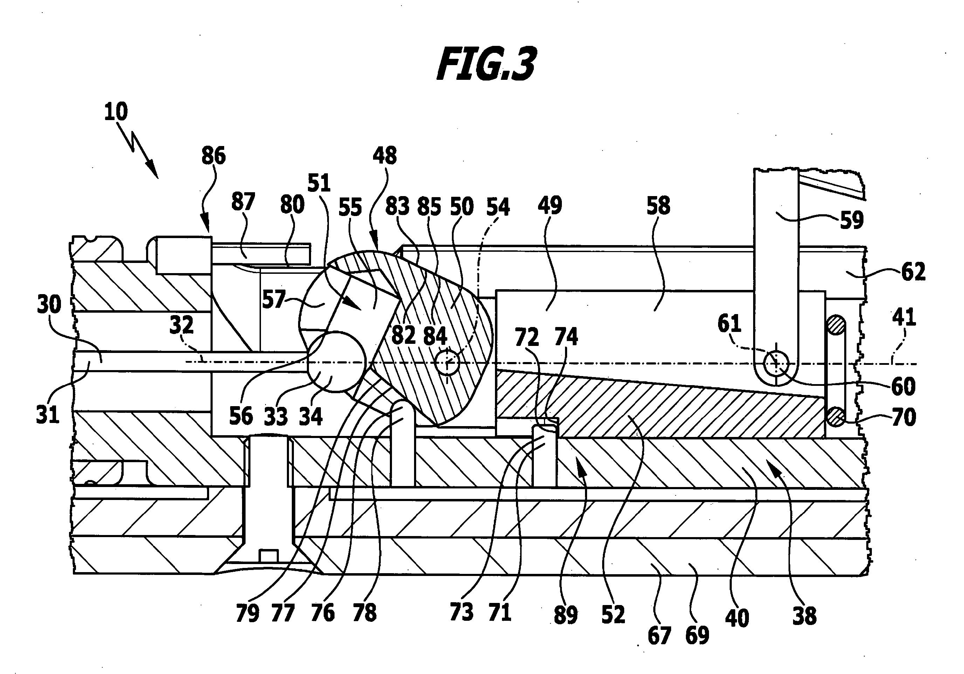

Die Verbindungselemente 46 sind Bestandteil einer Kopplungseinrichtung 48 des Handgriffs 10 zum Koppeln mit dem Rohrschaftwerkzeug 12, welche ein weiteres Kopplungselement 49 zum Koppeln mit der Kugel 34 der Stange 31 aufweist. Das Kopplungselement 49 ist im Innern der Hülse 40 aufgenommen, koaxial zu dieser ausgerichtet und kann von der Hülse 40 längs der Griffachse 41 verschoben und dabei von dieser geführt werden. Wie insbesondere aus Figur 3 deutlich wird, weist das Kopplungselement 49 distalseitig ein Aufnahmeglied 50 auf, in dem eine Aufnahme 51 für die Kugel 34 gebildet ist, sowie proximalseitig ein Bewegungsglied in Gestalt eines Verschiebegliedes 52, auf welches mittels einer Griffeinrichtung 53 des Handgriffs 10 eingewirkt werden kann. Das Aufnahmeglied 50 und das Verschiebeglied 52 sind relativ zueinander um eine quer zur Griffachse 41 ausgerichtete Ausrückachse 54 schwenkbar, wobei die Ausrückachse 54 und die Griffachse 41 eine Ebene aufspannen. Dabei greifen das Aufnahmeglied 50 und das Verschiebeglied 52 kettengliedartig ineinander, zu welchem Zweck proximalseitig am Aufnahmeglied 50 ein Vorsprung angeordnet ist, der in eine distalseitige schlitzförmige Aufnahme des Verschiebegliedes 52 eingreift.

An den proximalseitigen Vorsprung des Aufnahmegliedes 50 schließt sich distal ein kurzer zylindrischer Abschnitt desselben an, distalseitig von dem ein halbkugelförmiger Abschnitt des Aufnahmegliedes 50 angeordnet ist. Der halbkugelförmige und der zylindrische Abschnitt sind in Bezug auf die Griffachse 41 so bemessen, dass das Aufnahmeglied 50 spielfrei in der Hülse 40 aufgenommen und von dieser dadurch ebenfalls längs der Griffachse 41 verschieblich geführt sein kann.

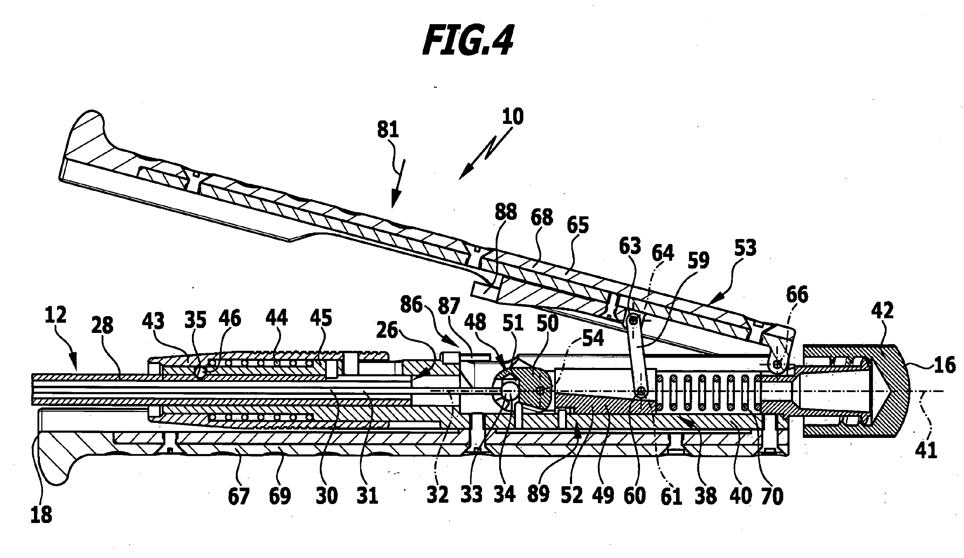

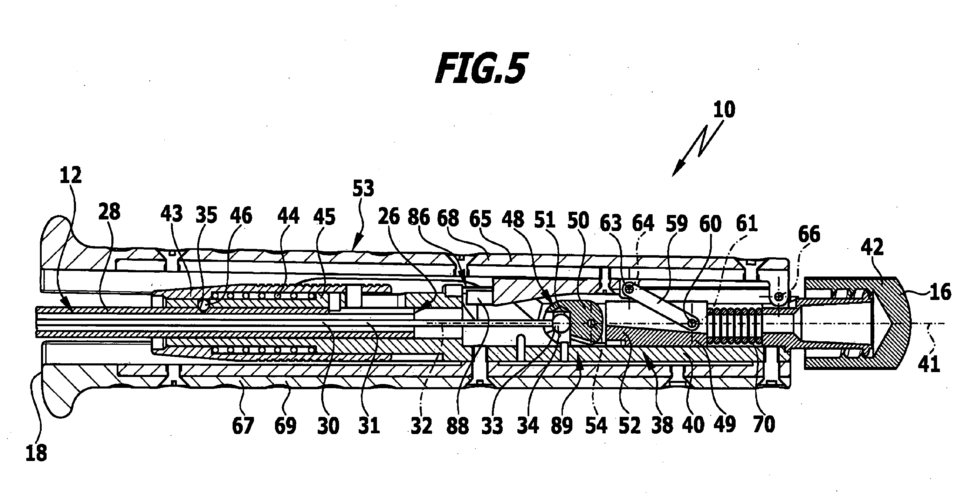

Am distalen Ende des Aufnahmegliedes 50 ist die Aufnahme 51 angeordnet, die ein quer zur Griffachse 41 ausgerichtetes Sackloch 55 aufweist mit einer Einführöffnung 56 für die Kugel 34. Eine proximale Seitenwand des Sacklochs 55 ist an einer Durchbrechung 57 axial geschlitzt. Ist die Kugel 34 im Sackloch 55 angeordnet, kann die Stange 31 die Durchbrechung 57 durchgreifen (Figuren 4 und 5), so dass eine auf das Kopplungselement 49 ausgeübte Kraft auf die Stange 31 und damit auf das Maulteil 24 übertragen werden kann.

Das Verschiebeglied 52 weist eine im Wesentlichen zylindrische Außenkontur auf, und es ist so bemessen, dass es von der Hülse 40 spielfrei umgeben und von dieser daher längs der Griffachse 41 verschieblich geführt sein kann. In axialer Richtung ist im Verschiebeglied 52 ein Längsschlitz 58 gebildet, in den distalseitig der proximale Vorsprung des Aufnahmegliedes 50 eingreift.

Proximalseitig greift in den Längsschlitz 58 ein unter einem Winkel zur Griffachse 41 ausgerichtetes Gelenkelement 59 ein, das die Wirkverbindung zwischen der Griffeinrichtung 53 und dem Kopplungselement 49 bereitstellt. Das Gelenkelement 59 ist an einem Gelenk 60 um eine Gelenkachse 61 schwenkbar mit dem Verschiebeglied 52 verbunden, wobei die Gelenkachse 61 in der vor der Griffachse 41 und der Ausrückachse 54 aufgespannten Ebene verläuft.

Das Gelenkelement 59 durchgreift eine schlitzförmige axiale Durchbrechung 62 der Hülse 40. Die Durchbrechung 62 erstreckt sich ungefähr über ein Drittel der Länge der Hülse 40, etwa ausgehend von deren proximalem Ende. Das Gelenkelement 59 durchgreift die Durchbrechung 62 im Wesentlichen spielfrei. Bei einer Relativbewegung der Hülse 40 und des Gelenkelementes 59 wird Letzteres daher in der Durchbrechung 62 geführt.

Das dem Verschiebeglied 52 gegenüberliegende Ende des Gelenkelementes 59 ist an einem Gelenk 63 um eine parallel zur Gelenkachse 61 verlaufende Gelenkachse 64 schwenkbar mit einem ersten Griffelement 65 der Griffeinrichtung 53 verbunden. Das erste Griffelement 65 ist seinerseits an einer Schwenkachse 66, die parallel zu den Gelenkachsen 61 und 64 ausgerichtet ist, schwenkbar an der Hülse 40 gelagert, wobei die proximalen Enden der Hülse 40 und des ersten Griffelementes 65 miteinander verbunden sind. Der Abstand der Schwenkachse 66 vom Gelenk 63 entspricht ungefähr einem Fünftel der Länge des ersten Griffelementes 65.

Beim Verschwenken des ersten Griffelementes 65 um die Schwenkachse 66 koppelt das Gelenkelement 59 das Griffelement 65 mit dem Kopplungselement 49, so dass abhängig vom Schwenkwinkel des Griffelementes 65 das Kopplungselement 49 axial relativ zur Hülse 40 verschoben wird. Das Gelenkelement 59 bildet daher einen Kopplungshebel zum Überführen der Schwenkbewegung des Griffelementes 65 in eine durch die Hülse 40 geführte translatorische Bewegung des Kopplungselementes 49 aus.

Über das erste Griffelement 65 hinaus weist die Griffeinrichtung 53 ein zweites Griffelement 67 auf. Die Griffelemente 65 und 67 sind jeweils schalenförmig ausgestaltet, wobei sie insbesondere die Gestalt von axial erstreckten, im Wesentlichen zylindrischen Halbschalen 68 bzw. 69 aufweisen. Die Halbschalen 68 und 69 nehmen ausgehend vom Stopfen 42 die übrigen Bauteile des Handgriffes 10 sowie das proximale Ende des Rohrschaftwerkzeuges 12 zwischen sich auf und sind bezogen auf die Griffachse 41 einander diametral gegenüberliegend angeordnet. In Längsrichtung des Handgriffes 10 erstrecken sich die Griffelemente 65 und 67 vom proximalen Ende der Hülse 40 bis zum distalen Ende 18.

Anders als das erste Griffelement 65 ist das zweite Griffelement 67 fest mit der Hülse 40 verbunden und relativ zu dieser unbeweglich. Es ist jedoch auch denkbar, dass auch das zweite Griffelement 67 beweglich ist und insbesondere schwenkbar mit der Hülse 40 verbunden ist. Beispielsweise könnte das zweite Griffelement 67 um eine parallel zur Schwenkachse 66 und quer zur Griffachse 41 ausgerichtete Schwenkachse schwenkbar an der Hülse 40 gelagert sein. Weiter ist denkbar, dass das zweite Griffelement 67 eine der Durchbrechung 62 diametral gegenüberliegende Durchbrechung der Hülse 40 durchgreift und mit dem Kopplungselement 49 über ein dem Gelenkelement 59 entsprechendes Gelenkelement in Wirkverbindung steht.

Die schwenkbare Verbindung der Hülse 40 und des ersten Griffelementes 65 miteinander am jeweiligen proximalen Ende ermöglicht es, dass das Griffelement 65 relativ zur Hülse 40 unterschiedlich weit abgespreizt werden kann. Der maximale Spreizwinkel ist durch die Länge des Gelenkelementes 59 gegeben, das mit dem Griffelement 65 und mit dem in der Hülse 40 gehaltenen Kopplungselement 49 verbunden ist.

Ausgehend von einer abgespreizten Stellung kann das Griffelement 65 um die Schwenkachse 66 verschwenkt und dadurch unter Verschiebung des Kopplungselementes 49 in proximaler Richtung der Hülse 40 angenähert werden und nahezu zur Anlage an dieser gelangen.

Zwischen dem Kopplungselement 49 und dem Stopfen 42 ist in der Hülse 40 ein elastisches Element in Gestalt einer Schraubenfeder 70 aufgenommen. Die Schraubenfeder 70 stützt sich distalseitig am Kopplungselement 49 und proximalseitig am Stopfen 42 ab, und entgegen der Wirkung der Schraubenfeder 70 kann das Kopplungselement 49 in proximaler Richtung verschoben werden, wenn die Griffeinrichtung 53 betätigt wird.

Nachfolgend wird auf das Ankoppeln der Stange 31 an das Kopplungselement 49 eingegangen sowie den bestimmungsgemäßen Gebrauch des Handgriffs 10 und des Rohrschaftwerkzeuges 12, wobei angenommen wird, dass der Rohrschaft 28 und die Stange 31 so weit wie möglich in die Hülse 40 hineingeschoben werden und dadurch die Werkzeugachse 32 und die Griffachse 41 in Übereinstimmung gebracht sind.

Zum Ankoppeln an die Stange 31 wird das Kopplungselement 49 so weit wie möglich in distaler Richtung verschoben. Dies erfolgt beispielsweise dadurch, dass ein Benutzer das erste Griffelement 65 relativ zur Hülse verschwenkt und mit seinem distalen Ende so weit wie möglich von dieser abspreizt. Ergänzend und/oder alternativ beaufschlagt die Schraubenfeder 70 das Kopplungselement 49 mit einer Kraft in distaler Richtung, was auch zum Abspreizen des ersten Griffelementes 65 von der Hülse 40 führt. Insbesondere kann die Schraubenfeder 70 so ausgelegt sein, dass ohne am Handgriff 10 angekoppeltes Rohrschaftwerkzeug 12 unter der Wirkung der Federkraft das Griffelement 65 maximal von der Hülse 40 abgespreizt wird und das Kopplungselement 49 in distaler Richtung eine maximal vorgeschobene Stellung einnimmt.

Der maximale Spreizwinkel (Figuren 2 und 3) ist zum einen durch die Länge des Gelenkelementes 59 gegeben. Zum anderen sind an der Hülse 40 und am Verschiebeglied 52 zusammenwirkende Anschlagglieder 71 bzw. 72 vorgesehen, die den Verschiebeweg des Kopplungselementes 49 in distaler Richtung begrenzen. Wirken die Anschlagglieder 71 und 72 zusammen, sind im vorliegenden Fall die Gelenke 60 und 63 in einer gemeinsamen Ebene senkrecht zur Griffachse 41 ausgerichtet. Dies stellt sicher, dass das erste Griffelement 65 von der Hülse 40 nur so weit abgespreizt werden kann, dass die Orientierung des Gelenkelementes 59 relativ zum Griffelement 65 und zum Kopplungselement 49 nicht "umschlägt", wobei das Gelenk 60 in proximal-distaler Richtung maximal auf gleicher Höhe angeordnet ist wie das Gelenk 63.

Das Anschlagglied 71 ist ausgestaltet als quer zur Hülse 40 ausgerichteter und in dieser gehaltener Anschlagstift 73, und das Anschlagglied 72 ist ausgestaltet als distalseitig am Verschiebeglied 52 gebildeter Absatz 74 mit quer zur Griffachse 41 ausgerichteter Anschlagfläche.

Wird das erste Griffelement 65 wie vorstehend erwähnt relativ zur Hülse 40 gespreizt und/oder das Kopplungselement 49 unter der Wirkung der Schraubenfeder 70 verschoben, führt dies zum Überführen des Kopplungselementes 49 in eine Ankopplungsstellung. Die Ankopplungsstellung kann auch als Entkopplungsstellung bezeichnet werden. Dies ist dadurch bedingt, dass der Handgriff 10 zusammenwirkende Führungsglieder 76 und 77 umfasst, die beim Verschieben des Kopplungselementes 49 in distaler Richtung die Relativstellung des Aufnahmegliedes 50 und des Verschiebegliedes 52 beeinflussen. Das Führungsglied 76 ist ein an der Hülse 40 gehaltener Führungsstift 78 mit Orientierung quer zur Griffachse 41. Der Führungsstift 78 kann mit einer korrespondierenden Führungsfläche 79 zusammenwirken, die am Aufnahmeglied 50 gebildet ist und das Führungsglied 77 ausbildet. Wird dementsprechend das Kopplungselement 49 in distaler Richtung verschoben, bevor die Anschlagsglieder 71 und 72 miteinander koppeln, kann die Führungsfläche 79 am Führungsstift 78 entlanggleiten. Weil die Führungsfläche 79 schräg zur Griffachse 41 ausgerichtet ist, führt dies zu einem Verschwenken des Aufnahmegliedes 50 relativ zum Verschiebeglied 52 um die Ausrückachse 54, und zwar derart, dass das Aufnahmeglied 50 von der Griffachse 41 weg verschwenkt wird (Figuren 2 und 3).

Um dem Handgriff 10 eine möglichst kompakte Bauform zu verleihen, ist proximalseitig der axialen schlitzförmigen Durchbrechung 62 in der Hülse 40 eine fensterförmige Durchbrechung 80 gebildet. Die Durchbrechungen 80 und 62 münden ineinander, und sie sind auf derselben Seite der Hülse 40 angeordnet, wobei sich die Durchbrechung 80 in Umfangsrichtung der Griffachse 41 über einen größeren Winkel erstreckt. Das Aufnahmeglied 50 kann beim Verschwenken um die Ausrückachse 54 in die Durchbrechung 80 eingreifen und dadurch relativ zur Griffachse 41 ausgerückt werden, wobei die Hülse 40 zugleich möglichst kompakt gebaut werden kann.

In der Ankopplungsstellung ist die Einführöffnung 56 der Aufnahme 51 axial freigegeben (Figuren 2 und 3). Dies ermöglicht es, die Kugel 34 axial in die Einführöffnung 56 und damit in die Aufnahme 51 einzuführen und den Rohrschaft 28 zugleich mit dem Handgriff 10 zu verbinden. Da die Werkzeugachse 32 bereits in Übereinstimmung mit der Griffachse 41 gebracht ist, weisen die Stange 31 und das Kopplungselement 49 die korrekte Relativorientierung auf, so dass die Kugel 34 zuverlässig und zielsicher in die Aufnahme 51 eingeführt werden kann.

Zum Lösen des Rohrschaftwerkzeuges 10 vom Rohrschaftinstrument 14 kann die Kugel 34 in entsprechender Weise aus der Aufnahme 51 entfernt werden, weswegen die Ankopplungsstellung des Kopplungselementes 49, wie erwähnt, auch als "Entkopplungsstellung" bezeichnet wird.

Ausgehend von der Ankopplungsstellung kann das Kopplungselement 49 in eine Kopplungsstellung überführt werden, in der die Stange 31 am Kopplungselement 49 angekoppelt ist zum operativen Einsatz des Rohrschaftinstrumentes 14.

Zum Überführen des Kopplungselementes 49 in die Kopplungsstellung kann der Benutzer das erste Griffelement 65 relativ zur Hülse 40 verschwenken unter Verringerung des Spreizwinkels zwischen dem Griffelement 65 und der Hülse 40. Hierzu ist das Griffelement 65 mit einer auf das zweite Griffelement 67 gerichteten und durch einen Pfeil 81 symbolisierten Betätigungskraft zu beaufschlagen. Über die Kopplung durch das Gelenkelement 59 wird das Kopplungselement 49 infolgedessen längs der Griffachse 41 und von der Hülse 40 geführt in proximaler Richtung verschoben. Alternativ oder ergänzend kann ein Benutzer das Rohrschaftwerkzeug 12, etwa am Maulteil 24 und vorzugsweise mit geschlossenem Maulteil 24, mit einer in proximaler Richtung gerichteten Kraft beaufschlagen, die über die Kugel 34 an das Aufnahmeglied 50 zum Verschieben des Kopplungselementes 49 übertragen wird.

Beim Ankoppeln der Stange 31 wirken Führungsglieder 82 und 83 zusammen, die jeweils ausgebildet sind als schräg zur Griffachse 41 ausgerichtete Führungsflächen 84 bzw. 85. Die Führungsfläche 84 ist an der Hülse 40 proximalseitig der fensterförmigen Durchbrechung 80 an einem Rand der schlitzförmigen Durchbrechung 62 gebildet. Die Führungsfläche 85 ist am Aufnahmeglied 50 gebildet, und beide Führungsflächen 84 und 85 liegen dem Führungsstift 78 und der Führungsfläche 79 bezogen auf die Griffachse 41 diametral gegenüber.

Beim Verschieben des Kopplungselementes 49 in proximaler Richtung wirken die Führungsflächen 84 und 85 so zusammen, dass das Aufnahmeglied 50 relativ zum Verschiebeglied 42 um die Ausrückachse 54 verschwenkt wird, und zwar aus der Durchbrechung 80 heraus in Richtung der Griffachse 41. Dies führt dazu, dass die Einführöffnung 56 von der Griffachse 41 weggeschwenkt und axial blockiert wird, wobei zugleich die Kugel 34 gewissermaßen "automatisch" in die Aufnahme 51 hineingezogen wird, bis die Stange 31 die Durchbrechung 57 des Aufnahmegliedes 50 durchgreift.

Die Kopplungsstellung wird eingenommen, wenn die Einführöffnung 56 axial versperrt ist und das Aufnahmeglied 50 und das Verschiebeglied 52 axial zueinander ausgerichtet sind. Die Schraubenfeder 70 kann dabei die Relativposition des Kopplungselementes 49 und der Hülse 40 zueinander definieren, so dass das Kopplungselement 49 die Entkopplungsstellung vorzugsweise dann einnimmt, wenn die Schraubenfeder 70 in leicht gespanntem Zustand zwischen dem Kopplungselement 49 und dem Stopfen 42 angeordnet ist und in der Kopplungsstellung unter erhöhter Spannung steht (Figuren 2 bzw. 4). In der Kopplungsstellung ist der Rohrschaft 28, wie erwähnt, ebenfalls am Handgriff 10 fixiert.

Das in die Kopplungsstellung überführte Kopplungselement 49 kann eine Nichtbetätigungsposition einnehmen, der eine Nichtbetätigungsstellung der Griffeinrichtung 53, insbesondere des Griffelementes 65 zugeordnet ist. In der Nichtbetätigungsstellung ist das Griffelement 65 weiterhin von der Hülse 40 abgespreizt, aber um einen geringeren Winkel, als wenn das Kopplungselement 49 die Entkopplungsstellung einnimmt. In der Nichtbetätigungsposition des Kopplungselementes 49 ist das Maulteil 24 geöffnet.

Zum weiteren bestimmungsgemäßen Gebrauch des Handgriffs 10 und des Rohrschaftinstrumentes 14 kann die Griffeinrichtung 53, insbesondere das Griffelement 65, von der Nichtbetätigungsstellung in eine Betätigungsstellung überführt werden und zugleich das Kopplungselement 49 von der Nichtbetätigungsposition in eine Betätigungsposition. Zu diesem Zweck kann das Griffelement 65 weiter mit der Betätigungskraft 81 in Richtung auf das zweite Griffelement 67 beaufschlagt werden, so dass das Griffelement 65 weiter relativ zur Hülse 40 unter Verkleinerung des Spreizwinkels verschwenkt wird. Das Gelenkelement 59 überträgt die Schwenkbewegung auf das Kopplungselement 49, welches in proximaler Richtung relativ zur Hülse 40 und von dieser geführt verschoben wird. Die Verschiebung erfolgt entgegen der Rückstellkraft der Schraubenfeder 70. Die an das Kopplungselement 49 angekoppelte Stange 31 wird ebenfalls in proximaler Richtung verschoben und dadurch das Maulteil 24 geschlossen. Je nachdem, wie weit das Griffelement 65 relativ zur Hülse verschwenkt wird, kann es eine Mehrzahl von Betätigungsstellungen einnehmen, denen jeweils eine unterschiedliche Betätigungsposition des Kopplungselementes 49 relativ zur Hülse 40 zugeordnet ist. In unterschiedlichen Betätigungspositionen ist das Maulteil 24 unterschiedlich weit geschlossen oder geöffnet.

Das erste Griffelement 65 kann so weit relativ zur Hülse 40 verschwenkt werden, bis es in Längserstreckung nahezu an dieser anliegt und gemeinsam mit dem zweiten Griffelement 67 eine im Wesentlichen zylindrische Hülse ausbildet, die die Hülse 40 umschließt (Figur 5). In dieser Betätigungsstellung kann das Griffelement 65 fixiert werden. Zur Fixierung des Griffelementes 65 ist eine Fixiereinrichtung 86 vorgesehen, die als Rasteinrichtung ausgestaltet ist. Die Fixiereinrichtung 86 umfasst zusammenwirkende Rastglieder 87 und 88. Das Rastglied 87 ist distalseitig der Durchbrechung 80 an der Hülse 40 angeordnet und greift in proximaler Richtung etwas in die Durchbrechung 80 ein. Das Rastglied 88 ist radial innen am ersten Griffelement 65 festgelegt. Zum Verrasten und Entrasten kann das Rastglied 88 in zwei Richtungen relativ zum Rastglied 87 etwas quer zur Griffachse 41 bewegt werden, um die Rastglieder 87, 88 in Eingriff bzw. außer Eingriff zu bringen. Die Fixiereinrichtung 86 kann dadurch eine so genannte Umlaufsperre ausbilden.

Zum Entriegeln der Fixiereinrichtung 86 kann ein Benutzer das erste Griffelement 65 weiter mit der Betätigungskraft beaufschlagen und in Richtung der Hülse 40 verschwenken, so dass die Rastglieder 87, 88 außer Eingriff geraten.

In umgekehrter Weise kann die Griffeinrichtung 53 von der verriegelten Betätigungsstellung in die Nichtbetätigungsstellung überführt werden und zugleich das Kopplungselement von der Betätigungsposition in die Nichtbetätigungsposition. Dies erfolgt über die Verschwenkung des Griffelementes 65 relativ zur Hülse 40 hinaus insbesondere unter der unterstützenden Rückstellkraft der Schraubenfeder 70.

Bei den hier beschriebenen Bewegungen des Kopplungselementes 49 relativ zur Hülse 40 ist es vorteilhaft, dass die Hülse 40 das Kopplungselement in rein axialer Richtung führt. Unabhängig von der Betätigungsstellung der Griffeinrichtung 53 kann somit beim Handgriff 10 eine rein axiale Bewegung der Stange 31 sichergestellt werden. Dies erweist sich als vorteilhaft, weil auf die Stange 31 keine quer zur Werkzeugachse 32 und zur Griffachse 41 ausgerichteten Querkräfte wirken. Dadurch lässt sich der Handgriff 10 und das Rohrschaftinstrument 14 auf verbesserte Weise handhaben. Eine vom Benutzer an der Griffeinrichtung 53 aufzubringende Betätigungskraft kann möglichst gering gehalten werden, aufgrund fehlender "Verschwendung" der Betätigungskraft durch auftretende Querkräfte auf die Stange 31. Darüber hinaus kann unter Einsatz des Handgriffes 10 besonders feinfühlig gearbeitet werden, da sich die Betätigungskraft in Abwesenheit von Querkräften auf die Stange 31 unabhängig von der Betätigungsstellung der Griffeinrichtung 53 durch einen Benutzer besser dosieren lässt. Zudem kann der Verschleiß der Stange 31 in Abwesenheit von Querkräften möglichst gering gehalten werden. Dadurch erhöht sich die Lebensdauer des Rohrschaftwerkzeuges 12.

Weiterhin erweist sich die Ankopplung der Stange 31 an das Kopplungselement 49 bzw. die Entkopplung davon als besonders handhabungsfreundlich. Dies wird dadurch erzielt, dass das Kopplungselement 49 wie erläutert in die Ankopplungs- oder Entkopplungsstellung überführt werden kann, in der die Stange 31 ebenfalls ohne Auftreten von Querkräften rein axial in die Aufnahme 51 eingeführt bzw. aus dieser entfernt werden kann. Für eine handhabungsfreundliche Bedienung des Handgriffes 10 erweisen sich weiterhin die Führungsglieder 76, 77, 82 und 83 sowie die Anschlagglieder 71 und 72 als vorteilhaft, die gemeinsam mit der Hülse 40 eine Führungseinrichtung 89 des Handgriffes 10 ausbilden zum Führen des Kopplungselementes 49.

Günstig ist es weiterhin, dass zum Entkoppeln bzw. Ankoppeln der Stange 31 das Kopplungselement 49 längs der Hülse 40 in Gegenrichtung der Überführung von der Nichtbetätigungsposition in die Betätigungsposition zu bewegen ist. Dadurch lässt sich weitestgehend vermeiden, dass die Stange 31 unabsichtlich vom Kopplungselement 49 entkoppelt wird, da hierfür das Griffelement 65 entgegen der im normalen Gebrauch erforderlichen Betätigung auf Druck auf Zug betätigt wird.

Es kann vorgesehen sein, dass das Rohrschaftinstrument 14 ein chirurgisches mono- oder bipolares Instrument ist und dass am Handgriff 10 ein elektrisches Anschlusselement für mindestens eine elektrische Leitung vorhanden ist, um die Stange 31 und/oder den Rohrschaft 28 elektrisch zu kontaktieren. Alternativ oder ergänzend kann eine Öffnung zum Durchführen eines Anschlusskabels am Handgriff 10 vorgesehen sein, beispielsweise durch den Stopfen 42 hindurch, wobei der Stopfen 42 auch von der Hülse 40 lösbar sein kann, um deren proximales Ende freizugeben.

## Patentansprüche

1. Chirurgischer Handgriff (10) für ein chirurgisches Rohrschaftinstrument (14), wobei der Handgriff (10) eine Kopplungseinrichtung (48) zum Koppeln mit einem Rohrschaftwerkzeug (12) umfasst, welches einen Rohrschaft (28) und ein relativ dazu hin- und herbewegliches Kraftübertragungselement (30) aufweist, wobei die Kopplungseinrichtung (48) ein Kopplungselement (49) zum Koppeln mit dem Kraftübertragungselement (30) und ein Verbindungselement (46) zum Verbinden mit dem Rohrschaft (28) aufweist, wobei der Handgriff (10) weiter eine Griffeinrichtung (53) umfasst, die mit dem Kopplungselement (49) in Wirkverbindung steht und von einer Nichtbetätigungsstellung in zumindest eine Betätigungsstellung und umgekehrt überführbar ist unter Abstandsänderung des Kopplungselementes (49) und des Verbindungselementes (46) relativ zueinander durch Überführen des Kopplungselementes (49) von einer Nichtbetätigungsposition in zumindest eine Betätigungsposition, wobei der Handgriff (10) eine Griffachse (41) definiert und eine Führungseinrichtung (89) umfasst, die einen Führungskörper (38) zum Führen des Kopplungselementes (49) beim Überführen von der Nichtbetätigungsposition in die zumindest eine Betätigungsposition längs der Griffachse (41) aufweist unabhängig von der Stellung der Griffeinrichtung (53) beim Überführen von der Nichtbetätigungsstellung in die zumindest eine Betätigungsstellung, wobei der Führungskörper (38) als axiale Hülse (40) ausgestaltet ist und die Griffachse (41) definiert, wobei das Kopplungselement (49) beim Überführen von der Nichtbetätigungsposition in die mindestens eine Betätigungsposition in der Hülse (40) verschieblich gelagert ist, und wobei die Hülse (40) eine axiale schlitzförmige Durchbrechung (62) längs zumindest eines Abschnittes eines vom Kopplungselement (49) relativ zur Hülse (40) beim Überführen von der Nichtbetätigungsposition in die zumindest eine Betätigungsposition und umgekehrt zurücklegbaren Verschiebeweges aufweist, **dadurch gekennzeichnet, dass** die Hülse (40) eine fensterförmige Durchbrechung (80) aufweist, die in Umfangsrichtung der Griffachse (41) einen größeren Winkelbereich überdeckt als die schlitzförmige Durchbrechung (62).

2. Handgriff nach Anspruch 1, **dadurch gekennzeichnet, dass** die fensterförmige Durchbrechung (80) distalseitig der schlitzförmigen Durchbrechung (62) angeordnet ist.

3. Handgriff nach Anspruch 1 oder 2, **dadurch gekennzeichnet, dass** die schlitzförmige Durchbrechung (62) und die fensterförmige Durchbrechung (80) in Umfangsrichtung der Griffachse (41) auf derselben Seite der Hülse (40) angeordnet sind und/oder ineinander münden.

4. Handgriff nach einem der voranstehenden Ansprüche, **dadurch gekennzeichnet, dass** das Kopplungselement (49) quer oder schräg zur Griffachse (41) ausrückbar ist und im ausgerückten Zustand teilweise in die fensterförmige Durchbrechung (80) eingreift oder diese durchgreift zum Koppeln des Kopplungselementes (49) mit dem Kraftübertragungselement (30) oder Entkoppeln von diesem.

5. Handgriff nach einem der voranstehenden Ansprüche, **dadurch gekennzeichnet, dass** die Griffeinrichtung (53) ein erstes Griffelement (65) umfasst, das um eine quer zur Griffachse (41) ausgerichtete Schwenkachse (66) am Führungskörper (38) oder an einem mit dem Führungskörper (38) verbundenen Lagerkörper schwenkbar gelagert ist.

6. Handgriff nach Anspruch 5, **dadurch gekennzeichnet, dass** der Handgriff (10) ein Gelenkelement (59) umfasst, das an einem ersten Gelenk (63) um eine quer zur Griffachse (41) ausgerichtete erste Gelenkachse (64) schwenkbar mit dem ersten Griffelement (65) verbunden ist und an einem zweiten Gelenk (60) um eine quer zur Griffachse (41) ausgerichtete zweite Gelenkachse (61) schwenkbar mit dem Kopplungselement (49) verbunden ist.

7. Handgriff nach Anspruch 6, **dadurch gekennzeichnet, dass** das Gelenkelement (59) die schlitzförmige Durchbrechung (62) durchgreift.

8. Handgriff nach Anspruch 6 oder 7, **dadurch gekennzeichnet, dass** die Griffeinrichtung (53) ein zweites Griffelement (67) umfasst, das unbeweglich am Führungskörper (38) oder am Lagerkörper festgelegt ist, insbesondere dass das erste Griffelement (65) und/oder das zweite Griffelement (67) schalenförmig ausgestaltet sind und den Führungskörper (38) in Umfangsrichtung der Griffachse (41) zumindest bereichsweise umgeben.

9. Handgriff nach einem der voranstehenden Ansprüche, **dadurch gekennzeichnet, dass** das Kopplungselement (49) ein Bewegungsglied (52) umfasst, das vom Führungskörper (38) beweglich und insbesondere verschiebbar geführt ist, sowie ein mit dem Bewegungsglied (52) gekoppeltes Aufnahmeglied (50), das eine Aufnahme (51) für das Kraftübertragungselement (30) umfasst, und dass das Kopplungselement (49) von einer Kopplungsstellung in eine Entkopplungsstellung und umgekehrt überführbar ist, wobei in der Kopplungsstellung eine Einführöffnung (56) der Aufnahme (51) für das Kraftübertragungselement (30) axial blockiert und in der Entkopplungsstellung die Einführöffnung (56) axial freigegeben ist.

10. Handgriff nach Anspruch 9, **dadurch gekennzeichnet, dass** die Aufnahme (51) ein im Aufnahmeglied (50) gebildetes, quer zur Griffachse (41) orientiertes Sackloch (55) aufweist mit einer in axialer Richtung geschlitzten Seitenwand.

11. Handgriff nach Anspruch 9 oder 10, **dadurch gekennzeichnet, dass** das Bewegungsglied (52) und das Aufnahmeglied (50) relativ zueinander beweglich sind zum Überführen des Kopplungselementes (49) von der Kopplungsstellung in die Entkopplungsstellung und umgekehrt, insbesondere dass das Aufnahmeglied (50) und das Bewegungsglied (52) relativ zueinander um eine quer zur Griffachse (41) ausgerichtete Ausrückachse (54) schwenkbar sind.

12. Handgriff nach Anspruch 11, **dadurch gekennzeichnet, dass** die Ausrückachse (54) und die Griffachse (41) eine Ebene definieren.

13. Handgriff nach Anspruch 11 oder 12, **dadurch gekennzeichnet, dass** das Aufnahmeglied (50) zum Überführen des Kopplungselementes (49) von der Kopplungsstellung in die Entkopplungsstellung und umgekehrt von der Griffachse (41) weg bzw. auf die Griffachse (41) zu verschwenkbar ist.

14. Handgriff nach einem der voranstehenden Ansprüche, **dadurch gekennzeichnet, dass** das Kopplungselement (49) zumindest abschnittsweise zylindrisch oder im Wesentlichen zylindrisch ausgestaltet und koaxial zur Griffachse (41) ausgerichtet ist und/oder dass das Kopplungselement (49) zumindest abschnittsweise axial geschlitzt ausgebildet ist zum Eingreifen eines das Kopplungselement (49) mit der Griffeinrichtung (53) koppelnden Gelenkelementes (59).

15. Chirurgisches Rohrschaftinstrument (14), umfassend einen Handgriff (10) nach einem der voranstehenden Ansprüche sowie mindestens ein Rohrschaftwerkzeug (12), das mit dem Handgriff (10) zusammenwirkt.

## Claims

1. A surgical hand grip (10) for a surgical tubular shaft instrument (14), wherein the hand grip (10) comprises a coupling device (48) for coupling to a tubular shaft tool (12) which has a tubular shaft (28) and a force transmission element (30) that is movable back and forth relative thereto, wherein the coupling device (48) has a coupling element (49) for coupling to the force transmission element (30) and a connecting element (46) for connecting to the tubular shaft (28), wherein the hand grip (10) also comprises a gripping device (53) which is operatively connected to the coupling element (49) and is transferable from a non-actuating disposition into at least one actuating disposition and vice versa thereby changing the mutual relative spacing between the coupling element (49) and the connecting element (46) due to the transferral of the coupling element (49) from a non-actuating position into at least one actuating position, wherein the hand grip (10) defines a grip axis (41) and comprises a guiding device (89) which has a guiding body (38) for guiding the coupling element (49) along the grip axis (41) during the transfer from the non-actuating position into the at least one actuating position independently of the disposition of the gripping device (53) during the transfer from the non-actuating disposition into the at least one actuating disposition wherein the guiding body (38) is in the form of an axial sleeve (40) and defines the grip axis (41), wherein the coupling element (49) is mounted in displaceable manner in the sleeve (40) during the transfer from the non-actuating position into the at least one actuating position, and wherein the sleeve (40) has an axial slit-like through opening (62) along at least a section of a displacement path which is traversed by the coupling element (49) relative to the sleeve (40) during the transfer from the non-actuating position into the at least one actuating position, and vice versa, **characterized in that** the sleeve (40) has a window-like through opening (80) which extends over a larger angular range in the circumferential direction of the grip axis (41) than the slit-like through opening (62).

2. A hand grip in accordance with Claim 1, **characterized in that** the window-like through opening (80) is arranged on the distal side of the slit-like through opening (62).

3. A hand grip in accordance with Claim 1 or 2, **characterized in that** the slit-like through opening (62) and the window-like through opening (80) are arranged on the same side of the sleeve (40) in the circumferential direction of the grip axis (41) and/or merge into one another.

4. A hand grip in accordance with any of the preceding Claims, **characterized in that** the coupling element (49) can be disengaged transversely or at an angle with respect to the grip axis (41) and, in the disengaged state, partially engages in the window-like through opening (80) or pass through it for coupling the coupling element (49) to the force transmission element (30) or for decoupling it therefrom.

5. A hand grip in accordance with any of the preceding Claims, **characterized in that** the gripping device (53) comprises a first gripping element (65) which is mounted on the guiding body (38) or on a mounting body that is connected to the guiding body (38) such as to be pivotal about a pivotal axis (66) that is oriented transversely relative to the grip axis (41).

6. A hand grip in accordance with Claim 5, **characterized in that** the hand grip (10) comprises a linking element (59) which is connected to the first gripping element (65) at a first articulated joint (63) such that it is pivotal about a first joint axis (64) that is oriented transversely relative to the grip axis (41) and is connected to the coupling element (49) at a second articulated joint (60) such that it is pivotal about a second joint axis (61) that is oriented transversely relative to the grip axis (41).

7. A hand grip in accordance with Claim 6, **characterized in that** the linking element (59) can engage through the slit-like through opening (62).

8. A hand grip in accordance with Claim 6 or 7, **characterized in that** the gripping device (53) comprises a second gripping element (67) which is fixed immovably to the guiding body (38) or to the mounting body, in particular that the first gripping element (65) and/or the second gripping element (67) are shell-like and surround the guiding body (38) in the circumferential direction of the grip axis (41) at least in sections.

9. A hand grip in accordance with any of the preceding Claims, **characterized in that** the coupling element (49) comprises a moving member (52) which is guided by the guiding body (38) in a moveable and in particular displaceable manner and also a receptacle member (50) which is coupled to the moving member (52) and comprises a receptacle (51) for the force transmission element (30), and **in that** the coupling element (49) is transferable from a coupling disposition into an uncoupling disposition and vice versa, wherein an entry opening (56) of the receptacle (51) for the force transmission element (30) is axially blocked in the coupling disposition and the entry opening (56) is axially freed in the uncoupling disposition.

10. A hand grip in accordance with Claim 9, **characterized in that** the receptacle (51) comprises a blind hole (55) which is formed in the receptacle member (50) incorporating a side wall slit in the axial direction and which is oriented transversely relative to the grip axis (41).

11. A hand grip in accordance with Claim 9 or 10, **characterized in that** the moving member (52) and the receptacle member (50) are moveable relative to each other for transferring the coupling element (49) from the coupling disposition into the uncoupling disposition and vice versa, in particular that the receptacle member (50) and the moving member (52) are pivotal relative to each other about a disengaging axis (54) which is oriented transversely relative to the grip axis (41).

12. A hand grip in accordance with Claim 11, **characterized in that** the disengaging axis (54) and the grip axis (41) define a plane.

13. A hand grip in accordance with Claim 11 or 12, **characterized in that** the receptacle member (50) is pivotal away from the grip axis (41) or towards the grip axis (41) for transferring the coupling element (49) from the coupling disposition into the uncoupling disposition and vice versa.

14. A hand grip in accordance with any of the preceding Claims, **characterized in that** the coupling element (49) is cylindrical or substantially cylindrical at least in sections thereof and is oriented coaxially with the grip axis (41) and/or **in that** the coupling element (49) is slit axially at least in sections thereof for engagement of a linking element (59) coupling the coupling element (49) to the gripping device (53).

15. A surgical tubular shaft instrument (14) comprising a hand grip (10) in accordance with any of the preceding Claims and also at least one tubular shaft tool (12) which cooperates with the hand grip (10).

## Revendications

1. Poignée chirurgicale (10) pour un instrument chirurgical à corps tubulaire allongé (14), instrument
dans lequel la poignée (10) comprend un dispositif de couplage (48) pour le couplage à un outil à corps tubulaire allongé (12), qui comporte un corps tubulaire allongé (28) et un élément de transmission de force (30) pouvant coulisser en va-et-vient par rapport à ce corps tubulaire allongé,
dans lequel le dispositif de couplage (48) comporte un élément de couplage (49) pour le couplage avec l'élément de transmission de force (30), et un élément de liaison (46) pour assurer la liaison avec le corps tubulaire allongé (28),
dans lequel la poignée (10) comprend, par ailleurs, un dispositif de préhension (53), qui est en liaison active avec l'élément de couplage (49), et peut être transféré d'un emplacement de non actionnement à au moins un emplacement d'actionnement et inversement, en produisant une modification de l'espacement de l'élément de couplage (49) et de l'élément de liaison (46) l'un par rapport à l'autre par transfert de l'élément de couplage (49) d'une position de non actionnement à au moins une position d'actionnement,
dans lequel la poignée (10) définit un axe de poignée (41) et comprend un dispositif de guidage (89), qui comporte un corps de guidage (38) pour guider l'élément de couplage (49) lors du transfert de la position de non actionnement à ladite au moins une position d'actionnement le long de l'axe de poignée (41), indépendamment de l'emplacement du dispositif de préhension (53) lors du transfert de l'emplacement de non actionnement audit au moins un emplacement d'actionnement,
dans lequel le corps de guidage (38) est réalisé sous la forme d'une douille axiale (40) et définit l'axe de poignée (41),
dans lequel l'élément de couplage (49) lors du transfert de la position de non actionnement à ladite au moins une position d'actionnement est monté coulissant dans la douille (40),
et dans lequel la douille (40) présente un passage traversant axial en forme de fente (62) le long d'au moins un tronçon d'une course de coulissement pouvant être effectuée par l'élément de couplage (49) par rapport à la douille (40), lors du transfert de la position de non actionnement à ladite au moins une position d'actionnement et inversement,
**caractérisée en ce que** la douille (40) présente un passage traversant en forme de fenêtre (80), qui, dans la direction périphérique de l'axe de poignée (41), couvre une zone angulaire plus grande que le passage traversant en forme de fente (62).

2. Poignée selon la revendication 1, **caractérisée en ce que** le passage traversant en forme de fenêtre (80) est agencé du côté distal du passage traversant en forme de fente (62).

3. Poignée selon la revendication 1 ou la revendication 2, **caractérisée en ce que** le passage traversant en forme de fente (62) et le passage traversant en forme de fenêtre (80) sont agencés, dans la direction périphérique de l'axe de poignée (41), sur le même côté de la douille (40), et/ou débouchent l'un dans l'autre.

4. Poignée selon l'une des revendications précédentes, **caractérisée en ce que** l'élément de couplage (49) peut être désenclenché transversalement ou de manière oblique par rapport à l'axe de poignée (41), et, dans l'état désenclenché, s'engage partiellement dans le passage traversant en forme de fenêtre (80) ou traverse celui-ci pour assurer le couplage de l'élément de couplage (49) avec l'élément de transmission de force (30), ou le découplage de celui-ci.

5. Poignée selon l'une des revendications précédentes, **caractérisée en ce que** le dispositif de préhension (53) comprend un premier élément de préhension (65), qui est monté pivotant autour d'un axe de pivotement (66) orienté transversalement à l'axe de poignée (41) sur le corps de guidage (38) ou sur un corps de palier lié au corps de guidage (38).

6. Poignée selon la revendication 5, **caractérisée en ce que** la poignée (10) comprend un élément d'articulation (59) qui, au niveau d'une première articulation (63), est relié au premier élément de préhension (65) de manière pivotante autour d'un premier axe d'articulation (64) orienté transversalement à l'axe de poignée (41), et, au niveau d'une deuxième articulation (60), est relié à l'élément de couplage (49) de manière pivotante autour d'un deuxième axe d'articulation (61) orienté transversalement à l'axe de poignée (41).

7. Poignée selon la revendication 6, **caractérisée en ce que** l'élément d'articulation (59) traverse le passage traversant en forme de fente (62).

8. Poignée selon la revendication 6 ou la revendication 7, **caractérisée en ce que** le dispositif de préhension (53) comprend un deuxième élément de préhension (67), qui est fixé de manière non mobile au corps de guidage (38) ou au corps de palier, notamment **en ce que** le premier élément de préhension (65) et/ou le deuxième élément de préhension (67) sont d'une configuration en forme de coque, et entourent le corps de guidage (38) au moins partiellement dans la direction périphérique de l'axe de poignée (41).

9. Poignée selon l'une des revendications précédentes, **caractérisée en ce que** l'élément de couplage (49) comprend un organe de mouvement (52), qui est guidé de manière mobile, notamment de manière coulissante, par le corps de guidage (38), ainsi qu'un organe de logement d'accueil (50), qui est couplé à l'organe de mouvement (52) et comporte un logement d'accueil (51) pour l'élément de transmission de force (30), et **en ce que** l'élément de couplage (49) peut être transféré d'un emplacement de couplage à un emplacement de découplage et inversement, et **en ce qu'**à l'emplacement de couplage une ouverture d'introduction (56) du logement d'accueil (51) pour l'élément de transmission de force (30) est bloquée axialement, et à l'emplacement de découplage l'ouverture d'introduction (56) est dégagée axialement.

10. Poignée selon la revendication 9, **caractérisée en ce que** le logement d'accueil (51) comprend un trou borgne (55), qui est formé dans l'organe de logement d'accueil (50), orienté transversalement à l'axe de poignée (41) et présentant une paroi latérale fendue dans la direction axiale.

11. Poignée selon la revendication 9 ou la revendication 10, **caractérisée en ce que** l'organe de mouvement (52) et l'organe de logement d'accueil (50) sont mobiles l'un par rapport à l'autre pour le transfert de l'élément de couplage (49) de l'emplacement de couplage à l'emplacement de découplage et inversement, notamment **en ce que** l'organe de logement d'accueil (50) et l'organe de mouvement (52) peuvent pivoter l'un par rapport à l'autre autour d'un axe de désenclenchement (54) orienté transversalement à l'axe de poignée (41).

12. Poignée selon la revendication 11, **caractérisée en ce que** l'axe de désenclenchement (54) et l'axe de poignée (41) définissent un plan.

13. Poignée selon la revendication 11 ou la revendication 12, **caractérisée en ce que** l'organe de logement d'accueil (50), pour le transfert de l'élément de couplage (49) de l'emplacement de couplage à l'emplacement de découplage et inversement, peut pivoter de manière à s'éloigner de l'axe de poignée (41) et respectivement vers l'axe de poignée (41).

14. Poignée selon l'une des revendications précédentes, **caractérisée en ce que** l'élément de couplage (49) est, au moins par tronçons, de configuration cylindrique ou sensiblement cylindrique et est orienté coaxialement à l'axe de poignée (41), et/ou **en ce que** l'élément de couplage (49) est, au moins par tronçons, réalisé axialement fendu pour la venue en prise d'un élément d'articulation (59) assurant le couplage de l'élément de couplage (49) avec le dispositif de préhension (53).

15. Instrument chirurgical à corps tubulaire allongé (14), comprenant une poignée (10) selon l'une des revendications précédentes, ainsi qu'au moins un outil à corps tubulaire allongé (12), qui interagit avec la poignée (10).
